Europäisches Patentamt

(19) European Patent Office

Office européen des brevets

(11) **EP 0 726 733 B1**

(12) **EUROPEAN PATENT SPECIFICATION**

(45) Date of publication and mention
of the grant of the patent:
**23.06.1999 Bulletin 1999/25**

(21) Application number: **92924361.6**

(22) Date of filing: **06.11.1992**

(51) Int. Cl.$^6$: **A61B 17/36**, A61B 1/00,
A61B 17/28, A61B 1/04

(86) International application number:
**PCT/US92/09616**

(87) International publication number:
**WO 94/10920 (26.05.1994 Gazette 1994/12)**

(54) **SURGICAL INSTRUMENT INCORPORATING FIBER OPTIC VIEWING SYSTEMS**

CHIRURGISCHES INSTRUMENT MIT EINEM FASEROPTISCHEN BETRACHTUNGSSYSTEM

INSTRUMENT DE CHIRURGIE PRESENTANT DES SYSTEMES D'OBSERVATION A FIBRES
OPTIQUES

(84) Designated Contracting States:
**BE CH DE FR GB IT LI NL**

(43) Date of publication of application:
**21.08.1996 Bulletin 1996/34**

(73) Proprietor:
**CLARUS MEDICAL SYSTEMS, INC.
Golden Valley, MN 55427 (US)**

(72) Inventors:
• **FINN, Miles, A.
Minneapolis, MN 55409 (US)**
• **POSS, Thomas, A.
Champlin, MN 55316 (US)**

• **RIEDL, Craig, L.
Long Lake, MN 55356 (US)**

(74) Representative:
**White, Martin Paul et al
Kilburn & Strode,
20 Red Lion Street
London WC1R 4PJ (GB)**

(56) References cited:
EP-A- 0 316 816          DE-A- 2 024 195
DE-U- 8 600 868          US-A- 4 300 564
US-A- 4 588 294          US-A- 4 759 348
US-A- 4 867 529          US-A- 5 147 356

## Description

BACKGROUND OF THE INVENTION

I. Field of the Invention:

This invention relates generally to surgical instruments, and more particularly to an improvement to conventional instruments which facilitates viewing of the surgical site by the surgeon from the perspective of the working end of that instrument.

II. Discussion of the Prior Art:

When performing many surgical procedures, surgeons require a clear view of the operating field and of the working end of their instruments so that the tissue structures to be cut and those to be preserved can be distinguished.

Various techniques have been developed to improve the visibility of the operating field. For example, surgical headlamps, typically mounted to a band worn on the physician's forehead, may be used to direct bright light in the direction in which the surgeon is looking. The beam from the headlamp can be used to provide illumination in an incision that would otherwise be in a shadow area if only the operating room overhead lights are being used. Another technique which has been used to improve visibility involves the use of loupes, which are binocular-like magnifying lenses adapted to be worn on a surgeon's glasses or on a headband. The magnification enables the surgeon to see details that would otherwise tend to be indistinguishable. Another device commonly used to improve visibility is the operating microscope. Such a device is designed such that there is a large working distance between the microscope's objective lenses and the surgical site under inspection. After an operating microscope is positioned over a patient, the surgeon can reach beneath the microscope and employ conventional surgical tools. Operating microscopes are available that provide two viewing systems, allowing an assisting surgeon to also view the surgical site along with the lead surgeon. Also, operating microscopes can be equipped with a video display system for allowing greater viewing participation and for recording.

In recent years, technology has advanced to the point where minimally invasive techniques can be used in carrying out an increasing variety of surgical procedures. Rather than making a large incision to expose the surgical site to view, various types of endoscopes are used which are designed to be inserted into the body through a normal orifice or through a substantially smaller puncture or incision. Rigid endoscopes, without tools or working channels, can be used strictly to monitor a surgical procedure. Typical of such devices are laparoscopes, such as used during laparoscopic cholecystectomy procedures. The laparoscope is inserted through a small puncture wound in the abdominal wall and various cannulae are likewise inserted, allowing cutting instruments and grasping instruments to be introduced through the lumen of these cannulae as the surgical field is being observed on a display device coupled to the laparoscope.

Still other types of rigid endoscopes are also known that have a working element or tool disposed at their distal ends. Such rigid endoscopes provide excellent optical images, but they suffer from several drawbacks. First, they require precise alignment and are therefore fairly expensive to manufacture. The optical system employed does not allow the endoscope to be bent and this necessarily limits the ability of the surgeon to gain access to many areas of the body to be worked on.

A flexible endoscope, on the other hand, provides access to parts of the body that are only accessible through tortuous, curved passages. By incorporating a working channel in a flexible endoscope, the surgeon may pass a flexible tool through the endoscope to manipulate tissue at the distal end of the endoscope. With a flexible endoscope, however, it becomes difficult to position the working end, when it is considered that the equal and opposite reaction to a force may easily push the end of a flexible endoscope away from the target tissue.

Flexible endoscopes offer a further advantage over rigid endoscopes in that with a flexible endoscope, it is possible to position a video camera in any convenient location whereas with a rigid endoscope, it must necessarily be suspended from the proximal end thereof. The additional weight of the camera makes it more difficult for the physician to manipulate the distal end of the endoscope to bring its lens system to a desired location and also seriously detracts from the tactile response provided by the instrument.

U.S. Patent 4,759,348 to Cawood describes an assembly of a flexible endoscope with a surgical instrument wherein only the endoscope's optical head is releasably clipped to the distal end portion of a surgical instrument, theoretically making it possible for the surgeon to view the working tip of the instrument. However, because it is intended that the clip-on head be secured to the surgical instrument at the time of the surgical procedure, obtaining proper alignment and focus becomes time consuming. Also, because the Cawood device only connects to the distal end portion of the surgical tool and its body does not follow or conform to the shape of the instrument, the weight of the flexible endoscope tends to dampen the tactile feel which is so important to a skilled surgeon. Moreover, a surgical device whose distal end cannot be precisely maneuvered tends to offend a surgeon's natural desire to maintain such control over that which can touch tissue. Furthermore, it presents yet another obstacle in the surgeon's field-or-view and requires a larger than necessary incision to allow entry of the endoscope and the surgical tool through the same opening.

EP-A-0,316,816 describes surgical tongs with an endoscope for viewing an operating area. The tongs include a shaft constructed from two elongated tubular elements in longitudinal alignment with each other. Attached to one end of the shaft is a tong bit with a moving part and a fixed part. The moving part is opened and closed by axial movement of the shaft elements relative to each other.

U.S. Patent 4,588,294 depicts a searching and measuring endoscope employing two image-transmitting systems in a common shaft. One system is fitted with a wide angle, fixed focus objective lens and the other system is fitted with a narrow angle, fixed focus objective lens.

It is accordingly a principal object of the present invention to provide a variety of improved surgical instruments incorporating one or more fiber optic viewing assemblies having a cross-sectional size allowing them to closely conform to the shape profile of the particular instrument employed and whose distal ends are accurately focused on the working element of the instrument.

Another object of the invention is to provide a variety of surgical instruments, such as used in arthroscopic, laparoscopic or other endoscopic procedures, but with each equipped with small diameter fiber optic viewing assemblies where the presence of the fiber optic viewing assemblies does not detract from the normal tactile feel presented to the surgeon as the instrument is being used in its normal, intended fashion.

Yet another object of the invention is to provide an improved surgical instrument with optical viewing capability in which the fiber optic bundle or bundles are operatively coupled to the instrument over a substantial length thereof such that the maneuverability of the instrument's distal end, having the working element thereon, is not compromised, nor is the surgeon's field-of-view obstructed.

According to the present invention there is provided a surgical instrument as claimed in the independent claim. Some preferred features are claimed in the dependent claims.

The foregoing features, objects and advantages of the invention are achieved by providing a variety of surgical instruments that have an elongated rigid shaft with working elements, such as scalpel blades, scissors blades, forceps, rongeurs, stitchers, curettes, or the like, affixed or otherwise disposed at the distal end of the shaft and a handle element affixed to its proximal end. In accordance with the invention, an elongated, flexible fiber-optic assembly, including at least one illumination fiber and a first image bundle comprising a first plurality of image fibers are secured to the rigid shaft along substantially the entire length thereof, with the distal ends of the illumination fiber and the plurality of image fibers optically aligned with a predetermined portion of the working element. Means are provided at the proximal end of the illumination fiber for allowing it to be coupled to a light source. Similarly, an appropriate device is affixed to the proximal end of the first image bundle, allowing it to be coupled to a viewing system, such as an eyepiece or a video camera and display.

The illumination fiber and the plurality of image fibers may be contained within a common sheath or, alternatively, may follow differing paths, each of which traverses substantially the entire length of the instrument's rigid shaft. By providing a second image bundle, also including a plurality of image fibers and which is secured to the rigid shaft of the instrument along substantially the entire length thereof with an objective lens at its distal end optically aligned with a predetermined portion of the instrument's working element, a view of the instrument's working element from multiple directions can be realized, thus aiding in the surgeon's depth perception and reducing "shadowing" where the working element blocks the view.

DESCRIPTION OF THE DRAWINGS

Figure 1 is a side elevation view showing the manner in which the present invention may be applied to a conventional surgical scissors;
Figure 2 is a cross-sectional view taken along the line 2-2 in Figure 1;
Figure 3 is a somewhat enlarged perspective view of the distal end portion of the instrument of Figure 1;
Figure 3A is a view like Figure 3, but of a rongeur or of a biopsy forceps;
Figure 4 shows the manner in which the present invention may be applied to a hook knife;
Figure 5 is a cross-sectional view taken along the line 5-5 in Figure 4;
Figure 6 is a cross-sectional view taken along the line 6-6 in Figure 4;
Figure 7 illustrates the invention applied to a surgical stitcher; and
Figure 8 is a distal end view of the device of Figure 7.

DESCRIPTION OF THE PREFERRED EMBODIMENT

As will become apparent to those skilled in the art from a reading of the following description, the present invention may be applied to various types of specialized medical instruments, including cutting and grasping instruments, surgical probes, stitchers, etc. However, the principles will be explained in connection with only a few specific instruments, namely, a surgical scissors, a rongeur, a hook knife and a stitcher.

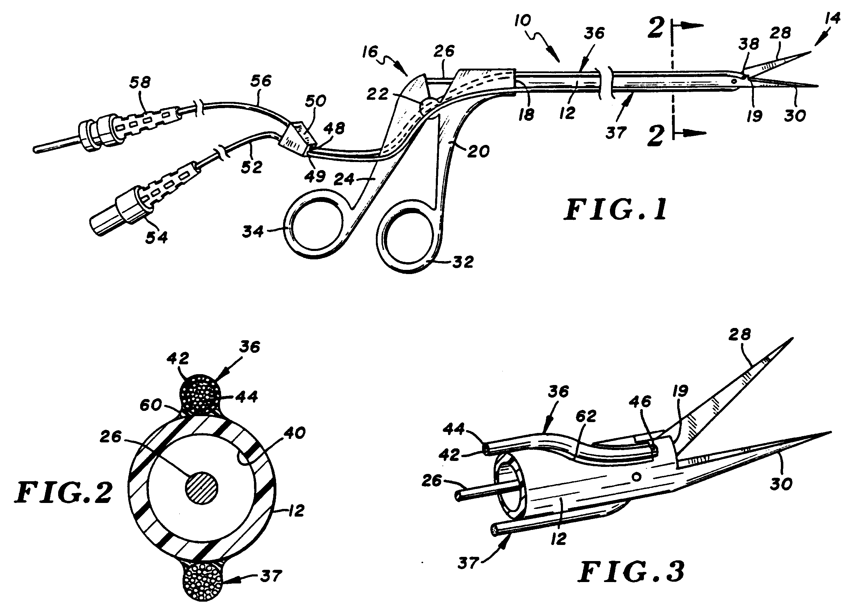

Referring to Figure 1, there is indicated generally by numeral 10, a surgical scissors that may find use in minimally invasive procedures, such as a laparoscopic cholecystectomy. The scissors 10 includes an elongated shaft 12 whose outside diameter is such that it can be passed through an introducer, a cannula or directly into a surgical incision and of a length allowing its working element (blades) 14 to reach the organ or tissue to be cut when the scissors handle member 16 affixed to its proximal end is manipulated. The rigid shaft 12 is tubular and its proximal end 18 is fitted into a bore formed in a first handle member 20 that is offset from the shaft 12. Pivotally joined to the first handle member 20 at pivot 22 is a second handle element 24. An actuating rod or cable 26 is appropriately affixed to the upper end of the second handle member 24 and it extends through the lumen of the tubular shaft 12 to join the movable blade 28 of the working element 14. The remaining blade 30 is preferably rigidly secured to the distal end 19 of the rigid shaft 12. By grasping the scissors handle members 20 and 24 by their respective finger-receiving loops 32 and 34 and by pivoting the handle member 24 back and forth relative to the stationary handle half 20, the rod or cable 26 moves reciprocally within the lumen of the rigid tube 12 to cause the movable blade half 28 to open and close in a scissors-like action relative to the stationary blade 30.

The surgical scissors, as thus-far described, is entirely conventional. In accordance with the present invention, however, at least one and preferably two elongated, flexible, fiber-optic assemblies, indicated generally by numerals 36 and 37 are affixed to and routed along substantially the entire length of the elongated rigid shaft 12, such that their distal ends, such as end 38 of assembly 36, is appropriately positioned to allow viewing of the surgical site from the perspective of the scissors blades. (The distal end of fiber-optic bundle 37 is hidden from the view in Figure 1.) By providing two image bundles whose objective lenses are focused on the working element of the instrument from two different perspectives, better depth perception and reduced shadowing can be attained.

As is shown in the cross-sectional view of Figure 2, the actuating rod or cable 26 passes through the lumen 40 of the tube 12 and affixed to the exterior wall of the shaft 12 are the fiber-optic assemblies 36 and 37. Fiber-optic assemblies suitable for use in practicing the present invention may be of the super-thin type disclosed in the Utsumi et al. U.S. Patent 4,867,529. The outside diameter of such "fiberscopes" can be less than about 0.5 mm. Assembly 36 would typically include at least one, but preferably several, illumination fibers 42 and a large plurality, e.g., 10,000, image fibers 44 to provide 10,000 pixel resolution. The image fibers may be about 10 microns in diameter are fused together at each end to thereby preserve their positional relationship relative to one another throughout all or a portion of their entire length of the fiber-optic assembly. The illumination fiber(s) and the plural image fibers may be contained within a common sheath as shown in bundle 36 or, alternatively, the illumination fiber(s) may be routed independently of the image fiber bundle(s). Since the bundle 36 includes an illumination fiber, it is not essential that bundle 37 also include one, provided the fiber(s) 42 provide adequate illumination.

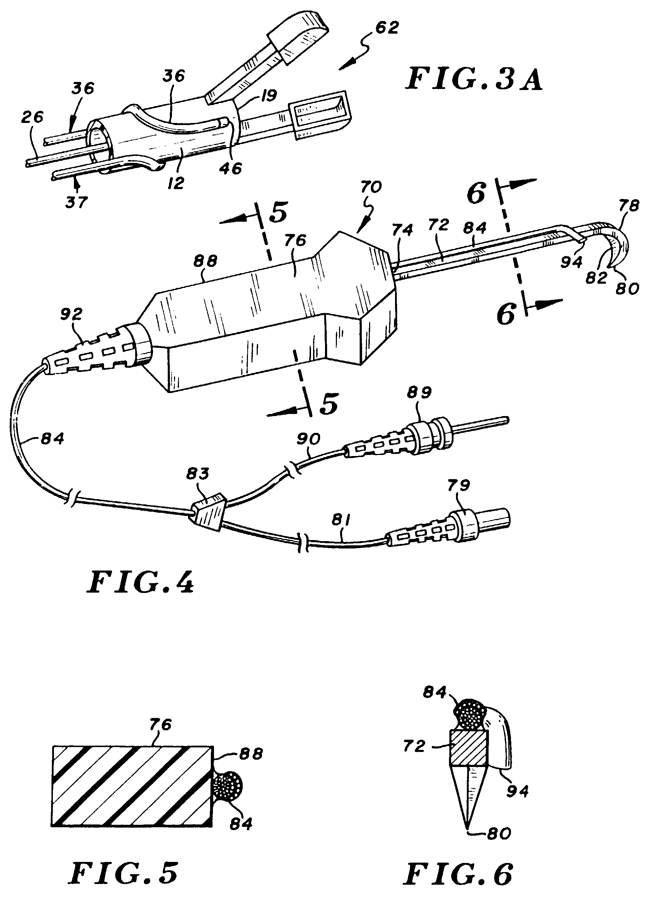

Referring to the enlarged partial view of the distal end portion of the instrument of Figure 1 illustrated in Figure 3, it is to be noted that an objective lens 46 is affixed to each of the distal ends of the fiber optic bundles 36 and 37 to focus the light rays reflected from the illuminated surgical site onto the plane occupied by the distal ends of all of the image fibers contained within the bundles 36 and 37.

The proximal ends 48 and 49 of the fiber optic assemblies 36 and 37 enter a molded plastic hub member 50 where the illumination fibers 42 are separated from the plurality of image fibers and then brought out through a protective sheath 52 to a connector 54 which is adapted to be connected to a light source. Likewise, the plurality of image fibers are brought out through the hub 50 and through a protective sheath 56 to an appropriate connector 58 which is designed to mate with a viewing system. The viewing system may, in its simplest form, comprise an eyepiece (not shown), which provides direct viewing of the image focused upon the distal end of the image fibers by the objective lens 46. However, the image present at the proximal connector 58 may also be fed to a video camera whose output is transmitted to a viewing screen for indirect observation by the surgeon and the surgical support staff.

It is desirable that the eyepiece or electronic viewing system provide appropriate magnification of the image. This will allow a surgeon to see objects that are otherwise difficult to see with the naked eye. An optical system designed for use in the present invention will have a field-of-view defined by a cone whose apex angle, $\Theta$, is in the range of from 50° to 70° when the object being viewed is in an air environment and a range of 37° to 52° if the object is immersed in a saline solution. It will provide a good image of the object placed about 5 mm from the objective lens. A video system used with the above-described optical arrangement will generate an image on the cathode ray television monitor and the diameter of that image will be approximately D = 5 in. (127 mm) or greater. The magnification can then be computed using the formula:

$$M = ((D/2)/(L\tan\Theta/2))$$

where D is the size of the image desired on the CRT screen, L is the distance between the objective lens and the object and $\Theta$ is a measure of the wideness of the field of view. Using the above formula with the numbers indicated for the various parameters, the magnification, M, to be used is a minimum of 26 times and a maximum of 38 times.

The fiber-optic bundles 36 and 37 may be affixed to the rigid shaft 12 by an appropriate bonding agent, such as epoxy, 60 (Figure 2), in which event the bonding would take place at the manufacturing facility of the instrument rather than in the operating room just prior to use.

Figure 3A shows the manner in which a fiber-optic viewing assembly may be added to a conventional rongeurs forceps. Here, the tubular shaft 12 has cup-like jaws 62 affixed to the distal end thereof and appropriately hinged and connected to an actuator rod 26, the same as in the embodiment of Figure 1. The fiber-optic assemblies 36 and 37 are again routed along substantially the entire length of the shaft 12 with the objective lens 46 arranged along an optical axis so as to be able to view the particular bone or other tissue to be grasped by the cup-shaped forceps paddles 62 when the scissors-style handle of the type shown in Figure 1 is manipulated.

Referring next to Figure 4, there is illustrated the manner in which the present invention may be applied to a surgical hook knife to thereby permit viewing of the nerve, ligament or other tissue to be severed. The hook knife itself is indicated generally by numeral 70 and is conventional. It includes an elongated rigid shaft 72 having a generally rectangular cross-section as best seen in the cross-sectional view of Figure 6. The shaft has a proximal end 74 fitted into a handle member 76 so that it can be conveniently and comfortably grasped by the surgeon. The shaft 72 is preferably made from stainless steel and its distal end is curved as at 78, terminating in a point 80 and having a beveled cutting edge 82 extending over the inward-facing arcuate portion of the hook.

The instrument of this type is generally used by inserting the shaft 72 of the knife through an opening until the arcuate blade 82 is disposed distally of the tissue to be severed. Cutting takes place by then pulling the knife back in the proximal direction which allows the cutting edge 82 to act upon the tissue to be severed. Carpal tunnel surgery is often performed using this type of instrument.

In accordance with the present invention, at least one fiber-optic assembly is affixed to the instrument in such a way that the surgical site can be viewed, either directly or indirectly, in the manner already described. In particular, a connector 79, which is adapted to mate with a light source, includes illumination fibers that pass through the sheath 81 into a connector hub 83. There they enter the common sheath surrounding the image fibers to form the fiber-optic bundle 84. If desired, the illumination fibers can also be routed separately from the image fibers and extend along side the bundle of image fibers.

Image connector 89 is joined to a plurality of image fibers within the sheath 90 which unite in the hub 82 with the illumination fibers in the fiber-optic bundle 84. This bundle passes through a flexible strain relief member 92 attached to the proximal end of the handle 76 of the hook knife.

The fiber-optic bundle 84 extends along a side surface 88 of the handle 76. Upon leaving the handle surface, bundle 84 is affixed to the exterior upper surface of the rectangular shaft 74. Near the distal end of the shaft, proximate the point where the shaft curves to form the hook, the fiber-optic bundle 84 wraps about the side surface of the shaft, such that its objective lens 94 becomes optically aligned with the knife edge 82. One observing the image exiting the proximal end of the image fibers in the fiber-optic assembly 84 obtains a remarkably clear view of the surgical site precisely where the blade interacts with the tissue. This adds appreciably to the ability of the surgeon to locate and identify the tissue to be cut.

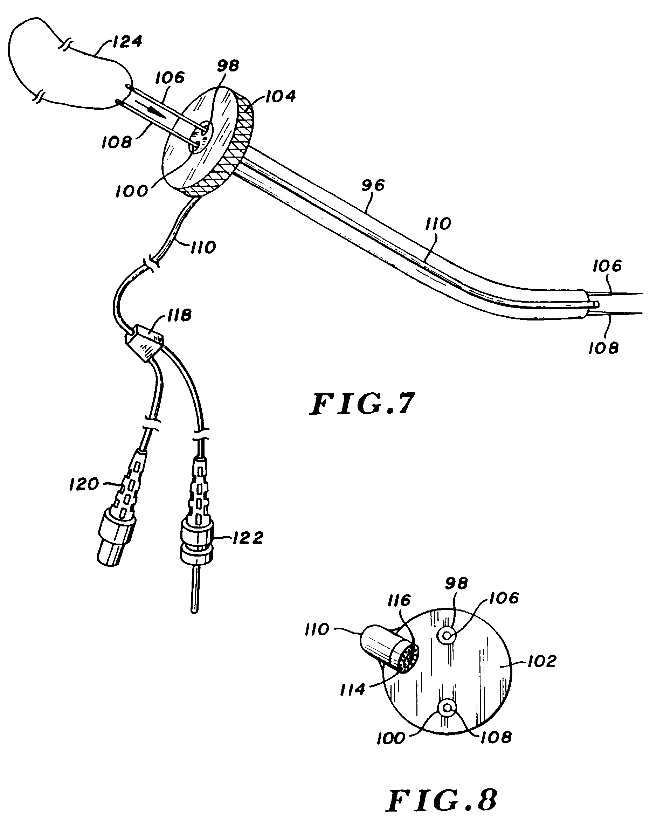

Figures 7 and 8 show the manner in which the present invention may be applied to an otherwise conventional surgical stitcher, such as the Acufex® Meniscal Stitcher, used in arthroscopic knee surgery. It is seen to comprise an elongated rigid tubular shaft 96 having lumens 98 and 100 extending from its proximal end to its distal end. Surrounding the proximal end of the shaft 96 is a knurled handle member 104. The lumens 98 and 100 accommodate stitching needles as at 106 and 108.

Affixed to the exterior of the shaft 96 is an optical fiber assembly 110 including an illumination fiber or bundle 114 and an image bundle 116 (Figure 8). Appropriately attached to the distal ends of the image fibers 116 is an objective lens positioned to view the distal end portions of needles 106 and 108 exiting the lumens in the shaft 96.

The illumination fibers are brought out through a hub member 118 to a connector 120, which is adapted to mate with a jack on a suitable light source (not shown). Similarly, the image fibers pass through the hub 118 and terminate in a connector 122 adapted to mate with the image input to a suitable viewing device, such as a video camera and associate video display.

In use, the rigid tubular shaft 96 acts as an introduction cannula and it is passed through a small surgical opening into the knee joint space so that its distal end can be brought up against the meniscus at a desired location. By viewing the image presented by the reflected light rays passing through the image fibers, the surgeon is able to precisely locate the distal end of the cannula at the desired site. The cannula is oriented so that the needles 106 and 108, having been inserted the entire length of the shaft 96, will pierce both sides of the tear in the meniscus to be repaired and will continue on through the capsule and soft tissues until the needle points pierce the skin opposite the entry portal. The needles, with a long strand of suture material 124 passing through their eyes, are then pulled out of the tissues and the loop of suture is drawn taut so as to complete the internal half of the stitch. A knot is then tied and drawn tight against the outer surface of the capsule through a small incision bridging the exit perforations of the needles. This procedure may be repeated as many times as necessary to effect repair of the tear.

The utility of the present invention can be appreciated upon considering a typical arthroscopic surgical procedure. In conventional arthroscopic surgery, it is necessary to make a plurality of punctures through the skin and into the capsule of a joint. Through this plurality of percutaneous punctures, also called "operating portals", tools are passed. One tool commonly used is a meniscus cutter. In conventional, prior art arthroscopic surgical procedures, the action of a meniscus cutter, passed through one of the operating portals, is observed with a separate instrument, namely, a rigid endoscope termed an arthroscope. It is passed through a second operating portal different from the one accommodating the meniscus cutter. One or more of a second type of percutaneous punctures, called "irrigation portals", may also be made through the skin and into the joint capsule. Irrigation fluid, which distends the joint and sweeps away blood clouded fluid, may be injected through and removed from the irrigation portals. While irrigation portals are generally of a small diameter, operating portals must necessarily be large enough to pass surgical tools and arthroscopes. It is, of course, desired to limit the number of operating portals required for surgery.

By using the instrument of the present invention, it is no longer necessary to use a separate rigid arthroscope in that the viewing optics would be adhered to the exterior surface of the meniscus cutter, thus obviating the need for a relatively large operating portal which would otherwise be used to accommodate that arthroscope.

It should be apparent to those skilled in the art that by appending the optical fiber assemblies to the surgical instruments along substantially the entire length of a rigid shaft portion of those instruments, the surgeon will still receive the desirable tactile response from the instrument. Also, better balance is retained than can be achieved when a conventional flexible endoscope is clipped only onto the distal end portion of the instrument as in the aforementioned Cawood '348 patent. Because the fiberoptic assemblies are made to conform to the profile of the instrument, it can be passed through a significantly smaller incision or through the lumen of a cannula or introducer, a result that cannot be attained with the arrangement shown in the Cawood patent.

The rigid shaft of the surgical instrument can include a groove in the exterior surface and at least a portion of the flexible fiber optic assembly is disposed in the groove. The rigid shaft can include a pair of grooves in the exterior surface and at least a portion of each of said first and second plurality of image fibers are disposed individually in the pair of grooves.

This invention has been described herein in considerable detail in order to comply with the Patent Statutes and to provide those skilled in the art with the information needed to apply the novel principles and to construct and use such specialized components as are required. However, it is to be understood that the invention can be carried out by specifically different equipment and devices, and that various modifications, both as to the equipment details and operating procedures, can be accomplished without departing from the scope of the invention itself.

**Claims**

1.   A surgical instrument (10) comprising:

(a) an elongated rigid shaft (12) having a proximal end (18) and a distal end (19);
(b) a working element (14,62) disposed at the distal end of the shaft (12) for manipulating tissue during the course of a surgical procedure;
(c) a handle element (16) affixed to the proximal end of the shaft (12) for facilitating the movement of the working element (14,62);
(d) an elongated flexible fiber-optic assembly; and
(e) means for securing the fiber-optic assembly to the exterior of the rigid shaft (12) along substantially the entire length thereof with the distal end (38) of the first plurality (36) of image fibers (44) positioned with a predetermined portion of the working element (14,62) within the field-of-view,
wherein the fiber optic assembly includes a first plurality (36) of image fibers (44) having a proximal end (48) and a distal end (38) with an objective lens (46) affixed to the distal end of the first plurality of image fibers for defining a predetermined field-of-view, and means are provided at the proximal end (48) of the first plurality (36) of image fibers (44) for coupling same to a viewing system;
characterised in that the fiber-optic assembly is made to conform to the profile of the instrument, including at least a portion of the handle element.

2.   The surgical instrument (10) as in claim 1 and further comprising at least one illumination fiber (42) secured to the exterior of the rigid shaft (12) along substantially the entire length thereof and the handle member (20) with the illumination fiber having a distal end that is positioned to project light on the predetermined portion of the working element (14,62).

3.   The surgical instrument (10) as in claims 1 or 2 and further including a second plurality (37) of image fibers having a proximal end (49) and a distal end with an objective lens affixed to the distal end of the second plurality of image fibers for defining another predetermined field-of-view with the ends of the second plurality of image fibers being disposed in viewing relation with a predetermined portion of the working element (14,62) along a viewing axis which is different from that of the first plurality of image fibers.

4. The surgical instrument (10) as in claim 3 and further including means for coupling the proximal ends (49) of the second plurality (37) of image fibers to the viewing system to provide multiple independent views of the predetermined portion of the working element (14,62).

5. The surgical instrument (10) as in claims 1, 2, 3, or 4 wherein the viewing system includes a video camera and means for displaying the image perceived by the video camera.

6. The surgical instrument (10) as in claims 3, 4 or 5 wherein the rigid shaft (12) includes a groove in the exterior surface and at least a portion of the flexible fiber optic assembly is disposed in the groove.

7. The surgical instrument (10) as in claims 3, 4 or 5 wherein the rigid shaft (12) includes a pair of grooves in the exterior surface and at least a portion of each of the first (36) and second (37) plurality of image fibers are disposed individually in the pair of grooves.

8. The surgical instrument (10) as in claims 1, 2, 3, 4, 5, 6, or 7 wherein the means for securing the assembly to the rigid shaft comprises a bonding agent, preferably an epoxy (60).

9. The surgical instrument (10) as in claims 1, 2, 3, 4, 5, 6, 7 or 8 wherein the at least one illumination fiber and the first plurality of image fibers follow different paths which traverse substantially the entire length of the rigid shaft (12).

10. The surgical instrument of claims 1, 2, 3, 4, 5, 6, 7, 8 or 9 wherein the working element (14,62) comprises a scissors blade (14) or rongeurs forceps (62).

11. The surgical instrument as claimed in any one of the preceding claims, where the handle element has a handle member (20) offset from the shaft.

**Patentansprüche**

1. Ein chirurgisches Instrument (10), umfassend:

    (a) einen verlängerten, steifen Schaft (12) mit einem proximalen Ende (18) und einem distalen Ende (19);
    (b) ein Arbeitselement (14, 62), das am distalen Ende des Schaftes (12) zur Behandlung von Gewebe während eines chirurgischen Eingriffes angeordnet ist;
    (c) ein Betätigungselement (16), das am proximalen Ende des Schaftes (12) zur Erleichterung der Bewegung des Arbeitselementes (14, 62) befestigt ist;
    (d) einen verlängerten, flexiblen faseroptischen Aufbau; und
    (e) Mittel zum Befestigen des faseroptischen Aufbaus an der Außenseite des steifen Schaftes (12) entlang im wesentlichen der gesamten Länge davon, wobei das distale Ende (38) der ersten Vielfalt (36) von Bildfasern (44) mit einem vorbestimmten Abschnitt des Arbeitselementes (14, 62) innerhalb des Betrachtungsfeldes angeordnet ist,
    worin der faseroptische Aufbau eine erste Vielzahl (36) von Bildfasern (44) mit einem proximalen Ende (48) und einem distalen Ende (38) umfaßt, wobei eine Objektivlinse (46) an dem distalen Ende der ersten Vielzahl von Bildfasern zur Begrenzung eines vorbestimmten Betrachtungsfeldes befestigt ist und Mittel am proximalen Ende (48) der ersten Vielzahl (36) von Bildfasern (44) vorgesehen sind, um diese mit einem Betrachtungssystem zu verbinden, dadurch gekennzeichnet, daß der faseroptische Aufbau mit dem Profil des Instrumentes übereinstimmend ausgebildet ist, einschließlich Wenigstens einem Teil des Betätigungselementes.

2. Chirurgisches Instrument (10) nach Anspruch 1, das des weiteren mindestens eine Beleuchtungsfaser (42) umfaßt, die an der Außenseite des steifen Schaftes (12) entlang im wesentlichen der gesamten Länge davon befestigt ist und das Betätigungselement (20) mit der Beleuchtungsfaser ein distales Ende aufweist, das angeordnet ist, um Licht auf den vorbestimmten Abschnitt des Arbeitselementes (14, 62) zu projezieren.

3. Chirurgisches Instrument (10) nach Anspruch 1 oder 2, das des weiteren eine zweite Vielzahl (37) von Bildfasern mit einem proximalen Ende (49) und einem distalen Ende umfaßt, wobei eine Objektivlinse an dem distalen Ende der zweiten Vielzahl von Bildfasern befestigt ist, um ein weiteres vorbestimmtes Betrachtungsfeld zu begrenzen, wobei die Enden der zweiten Vielzahl von Bildfasern in betrachtender Beziehung mit einem vorbestimmten Abschnitt des Arbeitselementes (14), 62) entlang einer Betrachtungsachse angeordnet ist, welche verschieden von jener der ersten Vielzahl von Bildfasern ist.

4. Chirurgisches Instrument (10) nach Anspruch 3, das des weiteren Mittel zur Verbindung der proximalen Enden (49) der zweiten Vielzahl (37) von Bildfasern mit dem Betrachtungssystem umfaßt, um mehrfach unabhängige Betrachtungen des vorbestimmten Abschnittes des Arbeitselementes (14, 62) vorzusehen.

5. Chirurgisches Instrument (10) nach den Ansprüchen 1, 2, 3 oder 4, worin das Betrachtungssystem eine Videokamera und Mittel zum Anzeigen des durch die Videokamera erfaßten Bildes umfaßt.

6. Chirurgisches Instrument (10) nach den Ansprüchen 3, 4 oder 5, worin der steife Schaft (12) eine Nut in der Außenoberfläche umfaßt, und mindestens ein Abschnitt des flexiblen, faseroptischen Aufbaus in der Nut angeordnet ist.

7. Chirurgisches Instrument (10) nach den Ansprüchen 3, 4 oder 5, worin der steife Schaft (12) ein Paar von Nuten in der äußeren Oberfläche umfaßt und mindestens ein Abschnitt einer jeden der ersten (36) und zweiten (37) Vielzahl von Bildfasern getrennt in dem Paar von Nuten angeordnet sind.

8. Chirurgisches Instrument (10) nach Anspruch 1, 2, 3, 4, 5, 6 oder 7, worin das Mittel zur Befestigung des Aufbaus an dem steifen Schaft ein Bindemittel, vorzugsweise ein Epoxid (60), umfaßt.

9. Chirurgisches Instrument (10) nach den Ansprüchen 1, 2, 3, 4, 5, 6, 7 oder 8, worin die wenigstens eine Beleuchtungsfaser und die erste Vielzahl von Bildfasern verschiedenen Wegen folgen, welche im wesentlichen die gesamte Länge des steifen Schaftes (12) überschreiten.

10. Chirurgisches Instrument nach den Ansprüchen 1, 2, 3, 4, 5, 6, 7, 8 oder 9, worin das Arbeitselement (14, 62) eine Scherenklinge (14) oder eine Greifpinzette (62) umfaßt.

11. Chirurgisches Instrument nach einem der vorhergehenden Ansprüche, worin das Betätigungselement ein Betätigungsglied (22) versetzt vom Schaft aufweist.

**Revendications**

1. Instrument chirurgical (10) comportant :

(a) un fût rigide allongé (12) présentant une extrémité arrière (18) et une extrémité avant (19) ;
(b) un outil (14, 62) disposé à l'extrémité avant du fût (12) pour manipuler les tissus au cours d'une opération chirurgicale ;
(c) une poignée (16) fixée à l'extrémité arrière (12) du fût pour faciliter le mouvement de l'outil (14, 62);
(d) un ensemble de fibres optiques flexible allongé ; et
(e) des moyens pour fixer l'ensemble de fibres optiques sur l'extérieur du fût rigide (12) le long de substantiellement toute sa longueur, l'extrémité avant (38) de la première pluralité (36) de fibres pour image (44) étant positionnée, avec une portion prédéterminée de l'outil (14, 62), à l'intérieur du champ visuel,
dans lequel l'ensemble de fibres optiques inclut une première pluralité (36) de fibres pour image (44) présentant une extrémité arrière (48) et une extrémité avant (38) avec une lentille objectif (46) fixée à l'extrémité avant de la première pluralité de fibres pour image pour définir un champ visuel prédéterminé, et dans lequel des moyens sont prévus à l'extrémité arrière (48) de la première pluralité (36) de fibres pour image (44) pour coupler ces fibres à un système de vision,
caractérisé par le fait que l'ensemble des fibres optiques est fait pour se conformer au profil de l'instrument, y compris à au moins une portion de la poignée.

2. L'instrument chirurgical (10) comme dans la revendication 1, et comportant en outre au moins une fibre pour éclairement (42) fixée à l'extérieur du fût rigide (12) le long substantiellement de toute sa longueur et de la poignée (20), la fibre pour éclairement ayant une extrémité avant qui est positionnée pour projeter de la lumière sur la portion prédéterminée de l'outil (14, 62).

3. L'instrument chirurgical (10) comme dans les revendications 1, et 2 et comportant en outre une seconde pluralité (37) de fibres pour image présentant une extrémité arrière (49) et une extrémité avant avec une lentille objectif fixée à l'extrémité avant de la seconde pluralité de fibres pour image pour définir un autre champ de vision prédéterminé, les extrémités de la seconde pluralité de fibres pour image étant disposées par rapport à une portion prédéterminée de l'outil (14, 62), dans une position de vision, le long d'un axe de vision différent de celui de la première pluralité de fibres pour image.

4. L'instrument chirurgical (10) comme dans la revendication 3, et comportant en outre des moyens pour coupler les extrémités arrière (49) de la seconde pluralité (37) de fibres pour image au système de vision pour donner de multiples vues indépendantes de la portion prédéterminée de l'outil (14, 62).

5. L'instrument chirurgical (10) comme dans les revendications 1, 2, 3 ou 4, dans lequel le système de vision inclut une caméra vidéo et des moyens pour afficher l'image perçue par la caméra vidéo.

6. L'instrument chirurgical (10) comme dans les revendications 3, 4 ou 5, dans lequel le fût rigide (12) inclut une rainure dans la surface extérieure et dans lequel au moins une portion de l'ensemble de fibres optiques flexible est disposée dans la rainure.

7. L'instrument chirurgical (10) comme dans les revendications 3, 4 ou 5 dans lequel le fût rigide (12) inclut une paire de rainures dans la surface extérieure et dans lequel, au moins une portion de chacune, de la première (36) et de la seconde (37) pluralités de fibres pour image, est disposée individuellement dans la paire de rainures.

8. L'instrument chirurgical (10) comme dans les revendications 1, 2, 3, 4, 5, 6 ou 7, dans lequel les moyens prévus pour fixer l'ensemble au fût rigide comportent un agent de fixation, de préférence un époxyde (60).

9. L'instrument chirurgical (10) comme dans les revendications 1, 2, 3, 4, 5, 6, 7 ou 8, dans lequel la fibre pour éclairement, dont il y a au moins une, et la première pluralité de fibres pour image suivent des chemins différents qui parcourent substantiellement toute la longueur du fût rigide (12).

10. L'instrument chirurgical des revendications 1, 2, 3, 4, 5, 6, 7, 8 ou 9, dans lequel l'outil (14, 62) comporte une lame de ciseaux (14) ou des forceps rongeurs (62).

11. L'instrument chirurgical (10) comme revendiqué dans l'une quelconque des revendications précédentes, dans lequel la poignée présente un élément (20) décalé par rapport au fût.

FIG.1

FIG.2

FIG.3

*FIG.3A*

*FIG.4*

*FIG.5*

*FIG.6*

*FIG.7*

*FIG.8*